# EUROPEAN PATENT SPECIFICATION

(11) **EP 2 231 862 B1**
(45) Date of publication and mention of the grant of the patent: **13.02.2013**
(21) Application number: 07870451.7
(22) Date of filing: 12.12.2007
(51) Int. Cl.: C12N 15/82, C12N 9/14, A01H 5/00

(54) **GENETICALLY MODIFIED PLANTS DISPLAYING REDUCED ACCUMULATION OF CADMIUM**
GENTECHNISCH VERÄNDERTE PFLANZEN MIT REDUZIERTER CADMIUMANREICHERUNG
PLANTES GÉNÉTIQUEMENT MODIFIÉES À ACCUMULATION RÉDUITE DE CADMIUM

(43) Date of publication of application: 29.09.2010
(73) Proprietor: Commissariat à l'Énergie Atomique et aux Énergies Alternatives, 75015 Paris (FR)
(72) Inventor: RICHAUD, Pierre, F-84120 La Bastidonne (FR); GRAVOT, Antoine, F-35590 L'hermitage (FR); AUROY, Pascaline, F-84360 Lauris (FR); VAVASSEUR, Alain, F-13090 Aix En Provence (FR)
(74) Representative: Rançon, Xavier Lucien Abel
(86) International application number: PCT/IB2007/004420
(87) International publication number: WO 2009/074843

(56) References cited:
- WO-A-02/081707
- VERRET F ET AL: "Overexpression of AtHMA4 enhances root-to-shoot translocation of zinc and cadmium and plant metal tolerance" FEBS LETTERS, ELSEVIER, AMSTERDAM, NL, vol. 576, no. 3, 22 October 2004 (2004-10-22), pages 306-312, XP004605705 ISSN: 0014-5793 cited in the application
- DUTTA SABARI J ET AL: "Conservative and nonconservative mutations of the transmembrane CPC motif in ZntA: Effect on metal selectivity and activity" BIOCHEMISTRY, vol. 46, no. 12, March 2007 (2007-03), pages 3692-3703, XP002495494 ISSN: 0006-2960
- WU CHEN-CHOU ET AL: "The cadmium transport sites of CadA, the Cd2+-ATPase from Listeria monocytogenes" JOURNAL OF BIOLOGICAL CHEMISTRY, vol. 281, no. 40, October 2006 (2006-10), pages 29533-29541, XP002495495 ISSN: 0021-9258
- VERRET FREDERIC ET AL: "Heavy metal transport by AtHMA4 involves the N-terminal degenerated metal binding domain and the C-terminal His11 stretch" FEBS LETTERS, vol. 579, no. 6, 28 February 2005 (2005-02-28), pages 1515-1522, XP002495496 ISSN: 0014-5793 cited in the application
- MILLS R F ET AL: "The plant P1B-type ATPase AtHMA4 transports Zn and Cd and plays a role in detoxification of transition metals supplied at elevated levels" FEBS LETTERS, ELSEVIER, AMSTERDAM, NL, vol. 579, no. 3, 31 January 2005 (2005-01-31), pages 783-791, XP004725196 ISSN: 0014-5793 cited in the application
- HUSSAIN DAWAR ET AL: "P-type ATPase heavy metal transporters with roles in essential zinc homeostasis in Arabidopsis" PLANT CELL, vol. 16, no. 5, May 2004 (2004-05), pages 1327-1339, XP002495497 ISSN: 1040-4651 cited in the application

## Description

The present invention relates to means and methods for reducing the accumulation of cadmium in a plant, in particular tobacco.

Large cultivated areas present rising cadmium concentrations. This is due on the one hand to the natural high level of metal in certain soils, and on the other hand to the use of fertilizers (due to the presence of cadmium in the phosphate rock used for their manufacture), the spread of wastewater treatment station sludge, the field watering with urban wastewaters and the laying of aerosols. In the near future, the cadmium concentration in cultivated soils should continue to strongly increase due to the depletion of cadmiumless-phosphate mines used as fertilizers. As a consequence, the cadmium content found in plants, mainly those cultivated for foodstuffs, could be more and more frequently over the standards implemented by the different countries or recommended by the Food and Agriculture Organization of the United Nations. By way of example, this is the case for green salad, tomato, pepper, wheat and, particularly, tobacco. Regarding tobacco, this plant is known to accumulate high level of cadmium in leaves; the cadmium concentration in tobacco is reported in the literature as being 0.5-5 ppm.

Cadmium is a metallic element belonging to group IIB of the periodic table of elements. Due to its chemical toxicity, inhalation or ingestion of high level of cadmium can represent severe health risks for both human and animals (including damage to the respiratory system, kidney or liver, and cancer).

Therefore, there is a need for plants that can grow in a soil containing high cadmium concentration, and that are able to have a low content of this harmful metal. While different strategies may be followed to reduce cadmium concentration in plants, the production of transgenic plant may also contribute to obtain such plants.

A method for producing transgenic plants with enhanced resistance and decreased uptake of cadmium has been proposed by Lee et al. (2003, Plant Physiol. 133:589-96; and International Application No. WO 02/081707). The authors have obtained genetically modified *Arabidopsis thaliana* expressing *Escherichia coli* heavy metal ZntA protein. The transgenic plants displayed improved resistance to Pb and Cd, and the shoots thereof had decreased Pb and Cd content compared to the wild type.

ZntA is a P_{1B}-type ATPase that confers to *E*. *coli* resistance to toxic concentrations of divalent cations, such as Zn²⁺, Cd²⁺ and Pb²⁺, by active efflux of these metal ions outside the cytoplasm (Rensing et al., 1997, PNAS, 94:14326-14331; Rensing et al., 1998, J Biol Chem., 273:32614-32617; Dutta et al., 2007, Biochemistry, 46:3692-3703).

P_{1B}-ATPases are transporters that use the energy liberated by the exergonic ATP hydrolysis reaction to translocate across membranes soft metal cations, such as Zn²⁺, Cd²⁺, Pb²⁺, Co²⁺, Cu⁺ and Ag⁺ (Inesi, 1985, Annu Rev Physiol., 47:573-601; Axelsen and Palmgren, 2001, Plant Physiol., 126:696-706; and for review: Argüello et al., 2007, BioMetals, 20:233-248). They are sometimes referred to as HMAs (for Heavy Metal ATPases) or to as CPx-type ATPases. P_{1B}-ATPases are found in prokaryote and eukaryote organisms, including archaea, bacteria, yeasts, insects, plants and mammals. They all contain the DKTGT signature amino acid sequence. P_{1B}-ATPases are divided into two main distinct subgroups (clusters) based on the substrate cation selectivity and phylogenetic analyses (Rensing et al., 1999, J Bacteriol., 181:5891-5897). The Cu⁺ cluster P_{1B}-ATPases are involved in the transport of Cu⁺ and Ag⁺, whereas the Zn²⁺ cluster P_{1B}-ATPases transport Zn²⁺ and other heavy metals such as Co²⁺, Cd²⁺ and Pb²⁺ (Axelsen and Palmgren, 2001, above-cited). The different subgroups of P_{1B}-ATPases have distinct motifs of conserved amino acid in their transmembrane domains. By way of example, their sixth transmembrane domain contains a (C, S, T)P(C,H) motif depending on the cation selectively transported (Solioz and Vulpe, 1996, Trends Biochem Sci., 21:237-241; Rensing *et al.,* 1998, above-cited; Williams et al., 2000, Biochim Biophys Acta., 1465:104-126; Dutta *et al.,* 2007, above-cited).

In *Arabidopsis thaliana,* HMA2 and HMA4 proteins (AtHMA2 and AtHMA4 respectively) are P_{1B}-type ATPases that cluster with the Zn²⁺/Co²⁺/Cd²⁺/Pb²⁺ subgroup. AtHMA2 and AtHMA4 are localized *in planta* at the plasma membrane and are involved in the transport of these divalent cations to the aerial parts of the plant (Hussain et al., 2004, Plant Cell., 16:1327-39, Verret et al., 2004, FEBS Lett., 576:306-312). Zinc is an essential micronutrient required by the plants, while cobalt, cadmium and lead exhibit toxic effects. The amino acid sequence of AtHMA2 and AtHMA4 are available under accession numbers GI|12229675 and GI|12229637 respectively in the GenBank database; AtHMA4 amino acid sequence is reproduced herein as SEQ ID NO: 1. These proteins contain the conserved CPC motif (herein after also denoted C₁PC₂) in their sixth transmembrane domain and the DKTGT motif in the soluble loop between their sixth and seventh transmembrane domain (Argüello *et al.,* 2007, above-cited). In AtHMA4, the first cysteine residue (C₁) is located at position 357 of the amino acid sequence (Mills et al., 2003, Plant J., 35:164-76). Mills *et al.* (2003, previously cited) showed that expression of AtHMA4 in *Saccharomyces cerevisiae* decreased the sensitivity of the yeast to Cd and conferred resistance to this metal ion. In a second article published in 2005 (FEBS Letters, 579:783-791), Mills *et al.* showed that expression in *S*. *cerevisiae* of an AtHMA4 mutant in which the first cysteine (C₁) of the CPC motif (³⁵⁷C) was substituted by a glycine (AtHMA4-C357G) did not confer Cd and Zn resistance to yeast, i.e., yeasts expressing AtHMA4-C357G were more sensitive to elevated levels of Cd and Zn than those expressing the wild type AtHMA4.

Transgenic *A. thaliana* overexpressing AtHMA4 have also been obtained (Verret *et al.,* above-cited; and International Application No. WO 2005/090583). Contrary to the transgenic *A. thaliana* expressing ZntA (*E. coli* P_{1B}-type ATPase that clusters the Zn²⁺ subgroup) obtained by Lee *et al.* (see above), the transgenic *A. thaliana* obtained by Verret *et al.* displayed an increase in the Zn and Cd shoot content compared to the wild type.

It emerges from the foregoing that expression or overexpression in a transgenic plant of a P_{1B}-type ATPase that clusters the Zn²⁺ subgroup can lead to a different phenotype according to the prokaryote or plant origin of the P_{1B}-type ATPase.

Within the framework of research that has lead to the present invention, the Inventors have found, unexpectedly, that *S*. *cerevisiae* expressing an AtHMA4 variant in which the first cysteine (C₁) residue of the conserved C₁PC₂ motif in the sixth transmembrane domain was substituted by a serine (AtHMA4-C357S), exhibited diminished Cd transport capabilities while the Zn efflux was almost identical in comparison with the wild type yeast. Then, the Inventors have shown that a transgenic *A. thaliana* expressing the AtHMA4-C357S variant displayed a reduced uptake of cadmium compared to the wild type, while, advantageously, homeostasis of the physiological Zn²⁺ micronutrient was unmodified.

Accordingly, in a first aspect, the present invention provides a method for reducing the accumulation of cadmium in the aerial parts of a plant without modifying the homeostasis of the physiological Zu²⁺ nutrient in said plant,, characterized in that said method comprises expressing in said plant a variant of a plant P_{1B}-type ATPase of the Zn²⁺/Co²⁺/Cd²⁺/Pb²⁺ subgroup having the C₁PC₂ motif in the sixth transmembrane domain, and localised *in planta* at the plasma membrane, said variant having a mutation consisting of the substitution of the C₁ residue by any amino acid selected from the group consisting of serine, alanine, histidine and threonine, preferably serine.

The term "aerial parts" includes, but is not limited to, the shoots, leaves, stems, flowers, fruits and seeds, preferably the leaves and seeds.

The localization of said P_{1B}-type ATPase *in planta* at the plasma membrane can be determined by methods well-known from one of ordinary skill in the art. By way of example, one can cite the methods described in Hussain *et al.,* 2004 and/or Verret *et al.,* 2004 (both above-cited). Briefly, total plasma membranes from plants are fractioned by aqueous two-phase partitioning and the fractions are characterized by protein gel blot analysis probed with P_{1B}-type ATPase specific antibodies. To confirm the plasma membrane localization of said P_{1B}-type ATPase, the coding sequence thereof can be fused in frame with a marker enzyme, such as the green fluorescent protein (GFP) and expressed under control of the constitutive promoter, such as the CaMV 35S promoter, in transgenic plants or in protoplasts (transient expression). The subcellular localization of the P_{1B}-type ATPase-marker enzyme in cells can then be visualized by confocal microscopy.

According to a preferred embodiment of the invention, said P_{1B}-type ATPase is from a higher plant, such as *A. thaliana, Nicotiana, Oryza* and *Populus,* and more preferably from the same plant species than the plant in which said expression is desired.

In another preferred embodiment, said P_{1B}-type ATPase is selected from the group consisting of HMA4 and HMA2 from *Arabidopsis thaliana,* HMA4 from *Thlaspi caerulescens* (available under accession number GI|46361990 in the GenBank database), HMA4 from *Arabidopsis halleri* subsp. *gemmifera* (available under accession number GI|63056225 in the GenBank database), HMA2 and HMA3 from *Oryza sativa* (available under accession numbers GI|125598398 and GI|125557764 respectively in the GenBank database) and HMA from *Vitis vinifera* (available under accession number GI|157357491 in the GenBank database).

According to another particular embodiment of the invention, said P_{1B}-type ATPase has at least 50% preferably at least 54% and by order of increasing preference, at least 56%, 60%, 65%, 70%, 75%, 80%, 85%, 90%, 95%, 97%, 98% and 99% sequence identity, or at least 70%, preferably 73% and by order of increasing preference, at least 79%, 80%, 85%, 90%, 95%, 97%, 98% and 99% sequence similarity with the AtHMA4 protein of SEQ ID NO: 1, provided that it contains the conserved C₁PC₂ motif in the sixth transmembrane domain.

Unless otherwise specified, the protein sequence identity and similarity values provided herein are calculated using the BLASTP program under default parameters, on a comparison window including the whole sequence of the proteins. Similarity calculations are performed using the scoring matrix BLOSUM62.

Optionally, the substitutions defined above can be combined with one or more other mutation(s) aiming at improving the activity of these mutants.

According to another particular embodiment of the invention, said variant has the amino acid sequence SEQ ID NO: 2. This amino acid sequence corresponds to the wild type AtHMA4 protein (SEQ ID NO: 1) in which the first cysteine (C₁) residue of the conserved C₁PC₂ motif in the sixth transmembrane domain of the protein is substituted by an amino acid as described above (*i.e*., serine, alanine, histidine or threonine).

According to another embodiment of the invention, the method for reducing the accumulation of cadmium in a plant further comprises the inhibition in said plant of at least one, preferably all, endogenous (wild type) P_{1B}-type ATPase(s) of the Zn²⁺/Co²⁺/Cd²⁺/Pb²⁺ subgroup having the C₁PC₂ motif in the sixth transmembrane domain and localized *in planta* at the plasma membrane.

The method according to this embodiment only concerns plants expressing said endogenous P_{1B}-type ATPase(s). Indeed, plants may express one or several endogenous P_{1B}-type ATPases as defined above. By way of example, *A. thaliana* expresses the P_{1B}-type ATPase HMA2 and HMA4. Therefore, when the method according to this embodiment is applied to *A. thaliana,* then the method comprises the inhibition of HMA2 and/or HMA4.

The inhibition of an endogenous P_{1B}-type ATPase can be obtained either by abolishing, blocking or decreasing its function, or advantageously, by preventing or down-regulating the expression of its gene.

By way of example, inhibition of said endogenous P_{1B}-type ATPases can be obtained by mutagenesis of the corresponding genes or of their promoters, and selection of the mutants having partially or totally lost the P_{1B}-type ATPase activity. For instance, a mutation within the coding sequence can induce, depending on the nature of the mutation, the expression of an inactive protein; in the same way, a mutation within the promoter sequence can induce a lack of expression of said endogenous P_{1B}-type ATPases, or decrease thereof.

Mutagenesis can be performed for instance by targeted deletion of the endogenous P_{1B}-type ATPase coding sequences or promoters, or of a portion thereof, or by targeted insertion of an exogenous sequence within said coding sequences or said promoter(s). It can also be performed by random chemical or physical mutagenesis, followed by screening of the mutants within the gene encoding said endogenous P_{1B}-type ATPases. Methods for high throughput mutagenesis and screening are available in the art. One can cite, for example, the method described in Hussain *et al.,* 2004 (above-cited) in which a T-DNA was inserted in the *hma2* and *hma4* alleles from *A. thaliana* and the mutants were grown on a medium containing high concentration of Zn (these mutants having Zn deficiency).

Advantageously, the inhibition of said endogenous P_{1B}-type ATPases is obtained by silencing of the corresponding genes. Methods for gene silencing in plants are known in themselves in the art. For instance, one can mention by antisense inhibition or co-suppression. It is also possible to use ribozymes targeting the mRNA of said endogenous P_{1B}-type ATPases.

Preferred methods are those wherein post transcriptional gene silencing is induced by means of RNA interference (RNAi) targeting the genes encoding said endogenous P_{1B}-type ATPases to be silenced. Various methods and DNA constructs for delivery of RNAi are available in the art (for review: Watson et al., 2005, FEBS Letters, 579:5982-5987).

Provided is a variant of a P_{1B}-type ATPase protein, as defined above.

The instant invention also provides means for carrying out said expression.

Thus, the present disclosure provides polynucleotides encoding a variant of a P_{1B}-type ATPase as defined above. The polynucleotides may be obtained by the well-known methods of recombinant DNA technology and/or of chemical DNA synthesis. These methods also allow to introduce the desired substitution in a naturally occurring nucleotide sequence encoding a P_{1B-}type ATPase as defined above.

Also provided are recombinant expression cassettes comprising a polynucleotide encoding a variant of a P_{1B}-type ATPase as defined above under control of a transcriptional promoter allowing the regulation of said polynucleotide in a host cell.

According to a preferred embodiment, said transcriptional promoter is any promoter functional in a plant cell, *i.e*., capable of directing transcription of a polynucleotide encoding a variant of a P_{1B}-type ATPase as defined above, in a plant cell. The choice of the more appropriate promoter may depend in particular on the organ(s) or tissue(s) targeted for expression, and on the type of expression (*i.e*., constitutive or inducible) that one wishes to obtain. A large choice of promoters suitable for expression of genes in plants, and in particular in tobacco, is available in the art. They can be obtained for instance from plants (*e.g., A. thaliana* and *N. tabacum*), plant viruses, or bacteria such as *Agrobacterium.* They include constitutive promoters, *i.e*. promoters which are active in most tissues and cells and under most environmental conditions, tissue or cell specific promoters which are active only or mainly in certain tissues (*e.g*., leaves) or certain cell types, and inducible promoters that are activated by physical or chemical stimuli. They also include the promoter of said endogenous P_{1B}-type ATPase from the same plant species than the plant in which said expression is desired. The sequence of the promoters can also be repeated two or more times (*e.g*., duplicated in tandem).

Non-limitative examples of constitutive promoters that are commonly used are the well-known cauliflower mosaic virus (CaMV) 35S promoter and the nopaline synthase (Nos) promoter, the Cassava vein Mosaic Virus (CsVMV) promoter (Verdaguer et al., 1996, Plant Mol. Biol., 31:1129-39), the rice actin (1 or 2) promoter followed by the rice actin intron (RAP-RAI) contained in the plasmid pAct1-F4 used for transgenic monocotyledon plants (McElroy et al., 1991, Mol. Gen. Genet., 231(1):150-160).

The expression cassettes generally also include a transcriptional terminator, such as the 35S transcriptional terminator or Nos terminator (Depicker et al., 1982, J. Mol. Appl. Genet., 1:561-73). They may also include other regulatory sequences, such as transcription enhancer sequences.

The recombinant expression cassettes can be inserted in an appropriate vector allowing genetic transformation of the genome of a host cell.

Thus, also provided are recombinant vectors containing an expression cassette as defined above, the promoter of said expression cassette being preferably a promoter functional in a plant cell.

The present invention also provides host cells containing a recombinant expression cassette or a recombinant vector as defined above.

The host cells of the present invention are eukaryotic cells, preferably plant cells, and more preferably tobacco cells.

In another aspect, the present invention provides a method for producing a transgenic plant having a reduced cadmium accumulation. Said method comprises the following steps:
a) providing a plant cell containing a recombinant expression cassette or a recombinant vector as defined above, and optionally in which the endogenous P_{1B}-type ATPase as defined above is inhibited, and
b) regenerating from the plant cell obtained in step a) a transgenic plant expressing a variant of a P_{1B}-type ATPase as defined above.

Owing to the use of a variant of a P_{1B}-type ATPase of plant origin, the method of the invention has the advantage that the transgenic plants produced contain a minimal content of xenogenetic elements or foreign sequences, which makes the expression of said variant more stable and more efficient.

The invention also comprises plants genetically transformed by a recombinant expression cassette of the invention, and expressing a variant of a P_{1B}-type ATPase as defined above. Preferably, said transgenic plants are obtainable by a method of the invention. In said transgenic plants, a recombinant expression cassette of the invention is comprised in one or several transgene(s) integrated (*i.e*., stably integrated) in the plant genome, so that it is passed onto successive plant generations. Thus the transgenic plants of the invention include not only the plants resulting from the initial transgenesis, but also their descendants, as far as they contain a recombinant expression cassette of the invention.

Advantageously, a transgenic plant of the invention expressing a variant of a P_{1B}-type ATPase as defmed above displays a reduced uptake of cadmium, particularly in the aerial parts, when compared with a plant of the same species devoid of said transgene.

Accordingly, the invention provides a transgenic plant or an isolated organ (such as seeds, leaves, flowers, roots, stems, ears, preferably leaves) or tissue thereof comprising, stably integrated in its genome, a recombinant expression cassette comprising a polynucleotide encoding a variant of a P_{1B}-type ATPase as defined above.

The present invention applies to monocot- or dicotyledon plants of agronomical interest, such as wheat, barley, corn, rape, rice, chard, spinach, lettuce, tomato, tobacco, preferably tobacco.

Tobacco leaves naturally accumulate and concentrate relatively high levels of cadmium, which is volatilized during burning, and contributes significantly to a smoker's exposure to cadmium. Advantageously, a tobacco leaf from a transgenic plant of the invention has lower cadmium content when compared to a wild type tobacco or a tobacco devoid of said transgene(s) defined above.

Accordingly, in another aspect, the present invention relates to the use a tobacco leaf from a transgenic plant of the invention for the manufacture of tobacco products including smoking products, such as cigarettes, cigars and shags, as well as smokeless products, such as snuffing, chewing, or sucking tobacco, and the like. Said products are also encompassed by the present invention.

Foregoing and other objects and advantages of the invention will become more apparent from the following detailed description and accompanying drawings. It is to be understood however that this foregoing detailed description is exemplary only and is not restrictive of the invention.

### Description of the drawings:

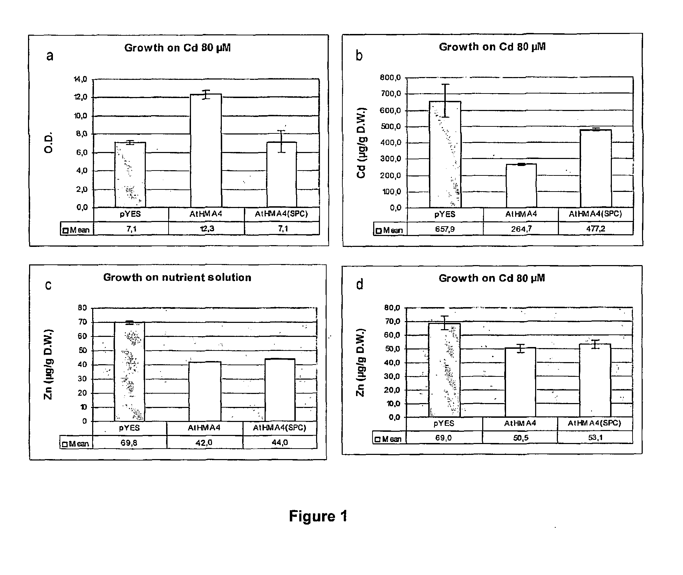

Figure 1: a) Growth of pYES-only, AtHMA4- and AtHMA4SPC-yeast transformants in liquid media followed by measuring optical density (OD) at 600 nm. Yeast cells were grown at 30°C for 48 h in the presence of Cd 80 µM. b-d) Liquid cultures were carried out in the same conditions as in a). The cells were collected, washed one time with 10 mM EDTA then washed two times with demineralised water to remove the cadmium adsorbed on the yeast cell walls. The pellets were dried, weighted, mineralised with HNO₃ then the metal content was determined by ICP-AES; in b) and d) the yeast cultures were carried out in the presence of Cd 80 µM; in c) the yeast cultures were carried out in a controlled nutrient solution (1.5 µM Zn).

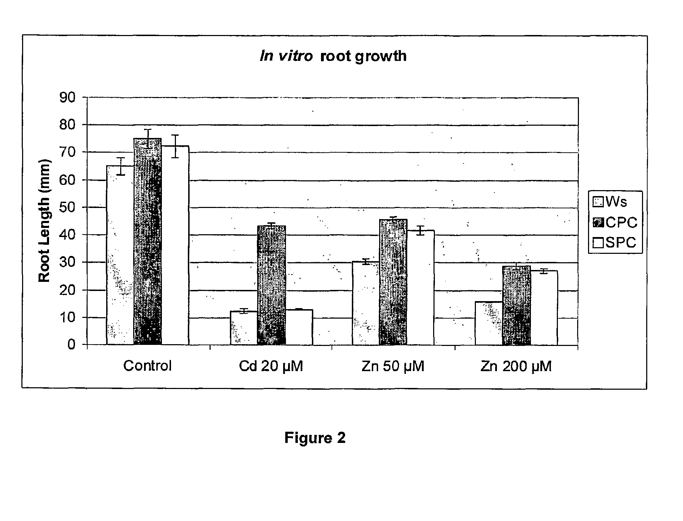

Figure 2 shows the *in vitro* root growth measurement of *A. thaliana* Wassilewskija (Ws) and transgenic *A. thaliana* overexpressing AtHMA4 (CPC) or AtHMA4SPC (SPC). Plantlets were grown vertically on bactoagar nutrient solution in the absence (control) or in the presence of the following metals, 20 µM Cd, 50 µM Zn or 200 µM Zn. The root length measurements were carried out 14 days after germination. The Figure indicates the mean values of 100-150 measures +/- SD.

### EXEMPLE: CHARACTERIZATION OF TRANSGENIC YEAST AND ARABIDOPSIS THALIANA EXPRESSING A VARIANT FORM OF AtHMA4

### 1) Material and Method:

The HMA4 cDNA from *Arabidopsis thaliana* (accession number AF412407 in the GENBANK database, Marsh 10, 2005 version), cloned in the inducible yeast vector pYES-GFP (Gravot et al., 2004, FEBS Lett., 561:22-28.), was modified by site-directed mutagenesis. The amino acid residue cysteine at position 357 in the sequence was substituted by a serine (clone pVF472) to obtain the amino acid sequence SEQ ID NO: 2 in which is Xaa is the amino acid serine (named AtHMA4SPC).

This construction was transformed in the yeast mutant strains hypersensitive to Cd and Pb (*ycf1*) (Li et al., 1996, J Biol Chem., 271:6509-6517) and to Zn (*zrc1*) (MacDiarmid et al., 2003, J Biol Chem., 278:15065-15072) respectively (clones pVF4121 and pVF4136, respectively) for heterologous experiments.

The insert of pVF472 was adapted by PCR for cloning in a plant vector presenting the strong ectopic promoter CaMV35S (clones pAP4152 and pAP4154). The vector pAP4154 was introduced in *Agrobacterium tumefaciens* strain AglI.

*A. thaliana* plants (ecotype Wassilewskija) were transfected by floral dip (Clough and Bent, 1998, Plant J., 16:735-743). The transformants were selected on hygromycin. Several independent lines of homozygous plants presenting one insertion of the construction were phenotypically characterized. Plants of wild type (Ws) and overexpressing the variant form of AtHMA4 (AtHMA4SPC) were grown on solid control or in a medium containing various cations transported by AtHMA4. Seeds were germinated in a controlled-environment (8 h photoperiod at 300 µmol m⁻² s⁻¹, 21 °C and 70 % relative humidity). The root length was measured 14 days after germination. T3 plants (homozygous plants carrying a unique T-DNA) were used for phenotypic characterization.

*A. thaliana* overexpressing AtHMA4 were obtained as described in Verret et al. 2004, FEBS Lett., 576:306-312.

### 2 Results:

### 2-1) Characterization of the transfected yeasts:

2-1-1) The wild type yeast *Saccharomyces cerevisiae* was transformed with the empty vector (pYES), the cDNA. encoding the wild type HMA4 from *A. thaliana* (AtHMA4, SEQ ID NO: 1), or the cDNA encoding a variant of AtHMA4 where the cystein at position 357 has been substituted with a serine (AtHMA4SPC, SEQ ID NO: 2). Liquid cultures in the presence of Cd 80 µM were carried out at 30°C and the O.D. was followed at 600 nm during 48 hours. In agreement with the results obtained by Verret et al. (2005, FEBS Lett., 579:1515-1522), a better growth of the yeast transformed with the wild type AtHMA4 was observed in the presence of Cd 80 µM. AtHMA4 induced an increased tolerance toward cadmium. When transformed with the variant form (AtHMA4SPC), the yeast did not present this increased tolerance and the growth was identical to the control strain. Results are shown in Figure 1a.

2-1-2) The metal contents in yeast were determined by ICP-AES as described by Gravot *et al.* (2004, above-cited). Results are shown in Figure 1b-d.

As previously described by Verret *et al.,* 2005 (above-cited), Cd concentration in yeast expressing AtHMA4 was found greatly lower (60 %) than the Cd concentration in the control strain (Figure 1b). AtHMA4 was expressed at the plasma membrane of yeasts and had a role in an ATP-dependent Cd/Zn efflux.

The Cd concentration in yeast expressing AtHMA4SPC was halfway between both other strains (27.5 % lower than the control). A lower capability to transport and efflux Cd could explain this observation.

The Zn concentration was measured in two growth conditions: in nutrient solution containing 1.5 µM Zn, and in the presence of Cd 80 µM. In both cases, the Zn concentration was identical in AtHMA4- and AtHMA4SPC-transformed yeasts and lower than in control yeasts (Figures 1c and 1d).

These experiments show that the Zn transport function of AtHMA4 was not modified by the C357S substitution, even in the presence of a toxic concentration of Cd, whereas the Cd transport capability was diminished.

### 2-2) Characterization of the transfected A. thaliana:

The plant tolerance toward the various metal transported by AtHMA4 or AtHMA4SPC was determined by the measure of the root length on bactoagar solid medium. The root length was the same for both genotypes in control condition. Results are shown in Figure 2.

In the presence of different concentrations of Zn, Co and Pb, the root length was greater for seedlings overexpressing AtHMA4SPC than for the wild type ones. These results were in agreement with the results obtained in yeast, and with the observations made by Verret *et al.* (above-cited) in plants overexpressing the native form of AtHMA4. Plants overexpressing the variant form of AtHMA4 (AtHMA4SPC) present, as the ones overexpressing the native form of AtHMA4, an increased tolerance toward these three metals. This is due to more important metal transport and translocation to their shoot capabilities.

In the presence of Cd 20 µM, the root length was the same for both lines (wild type and variant). The increased tolerance toward cadmium also observed for the plants overexpressing AtHMA4 was lost. This result is in agreement with those obtained with yeasts.

To conclude, a variant of a P_{1B}-type ATPase of the Zn²⁺/Co²⁺/Cd²⁺/Pb²⁺ subgroup of eukaryotic origin having the C₁PC₂ motif in the sixth transmembrane domain (e.g., AtHMA4), said variant having a mutation consisting of the substitution of the C₁ residue by a serine, presents a modified metal transport specificity probably due to a decreased affinity toward cadmium. However, this property is unchanged toward the other three transported metals Co, Pb and especially the physiological Zn.

## Claims

1. A method for reducing the accumulation of cadmium in the aerial parts of a plant without modifying the homeostasis of the physiological Zn²⁺ nutrient in said plant, **characterized in that** said method comprises expressing in said plant a variant of a plant P_{1B}-type ATPase of the Zn²⁺/Co²⁺/Cd²⁺/Pb²⁺ subgroup having the C₁PC₂ motif in the sixth transmembrane domain and localised *in planta* at the plasma membrane, said variant having a mutation consisting of the substitution of the C₁ residue by any amino acid selected from the group-consisting of serine, alanine, histidine and threonine.

2. The method according to claim 1, **characterised in that** said P_{1B}-type ATPase is from a higher plant.

3. The method according to claim 2, **characterized in that** said P_{1B}-type ATPase is from the same plant species than the plant for which said expression is desired.

4. The method according to any of claims 1 to 3, **characterized in that** said P_{1B}-type ATPase is selected from the group consisting of HMA4 and HMA2 from *Arabidopsis thaliana,* HMA4 from *Thlaspi caerulescens,* HMA4 from *Arabidopsis halleri* subsp. *gemmifera,* HMA2 and HMA3 from *Oryza sativa* and HMA from *Vitis vinifera.*

5. The method according to claim 4, **characterized in that** the variant of a P_{1B}-type ATPase has the amino acid sequence SEQ ID NO: 2.

6. The method according to any of claims 1 to 5, **characterized in that** it further comprises the inhibition of at least one endogenous P_{1B}-type ATPase of the Zn²⁺/Co²⁺/Cd²⁺/Pb²⁺ subgroup having the C₁PC₂ motif in the sixth transmembrane domain and localised *in planta* at the plasma membrane.

7. A plant cell, **characterized in that** it contains a recombinant expression cassette comprising a polynucleotide encoding a variant of a P_{1B}-type ATPase as defined in any of claims 1 to 5 under control of a transcriptional promoter or **in that** it contains a recombinant vector containing said recombinant expression cassette.

8. A plant cell according to claim 7, **characterized in that** it is a tobacco cell.

9. A method for producing a transgenic plant having a reduced cadmium accumulation while homeostasis of the physiological Zn²⁺ micronutrient is unmodified, **characterized in that** it comprises the following steps:
a) providing a plant cell of claim 7, and
b) regenerating from the plant cell obtained in step a) a transgenic plant expressing a variant of a P_{1B}-type ATPase as defined in any of claims 1 to 5.

10. The method according to claim 9, **characterized in that** the endogenous P_{1B}-type ATPase as defined in claim 6 is inhibited in said plant cell provided in step a).

11. A transgenic plant having a reduced cadmium accumulation while homeostasis of the physiological Zn²⁺ micronutrient is unmodified or an isolated organ thereof or tissue thereof, **characterized in that** it comprises, stably integrated in its genome, a recombinant expression cassette comprising a polynucleotide encoding a variant of a P_{1B}-type ATPase as defined in any of claims 1 to 5, under control of a transcriptional promoter.

12. A transgenic plant or an isolated organ thereof or tissue thereof according to claim 11, **characterized in that** the endogenous P_{1B}-type ATPase as defined in claim 6 is inhibited.

13. The transgenic plant according to claim 11 or claim 12, **characterized in that** it is a transgenic tobacco plant.

14. An isolated organ according to claim 11 or claim 12, **characterized in that** it is a tobacco leaf.

15. Use of a tobacco leaf of claim 14 for the manufacture of tobacco products.

## Patentansprüche

1. Verfahren zur Reduktion der Anhäufung von Cadmium in den oberirdischen Teilen einer Pflanze ohne die Homöostase des physiologischen Zn²⁺-Nährstoffs in der Pflanze zu verändern, **dadurch gekennzeichnet, dass** das Verfahren das in der Pflanze Exprimieren einer Variante einer Pflanzen P_{1B}-Typ ATPase der Zn²⁺/Co²⁺/Cd²⁺/Pb²⁺-Untergruppe, die das C₁PC₂-Motiv in der sechsten Transmembran-Domäne hat und *in planta* in der Plasmamembran lokalisiert ist umfasst, wobei die Variante eine Mutation hat, die aus der Substitution des C₁-Rest durch irgendeine Aminosäure, ausgewählt aus der Gruppe bestehend aus Serin, Alanin, Histidin und Threonin, besteht.

2. Verfahren gemäß Anspruch 1, **dadurch gekennzeichnet, dass** die P_{1B}-Typ ATPase von einer höheren Pflanze ist.

3. Verfahren gemäß Anspruch 2, **dadurch gekennzeichnet, dass** die P_{1B}-Typ ATPase von derselben Pflanzenart ist wie die Pflanze, für die die Expression gewünscht ist.

4. Verfahren gemäß einem der Ansprüche 1 bis 3, **dadurch gekennzeichnet, dass** die P_{1B}-Typ ATPase ausgewählt ist aus der Gruppe bestehend aus HMA4 und HMA2 von *Arabidopsis thaliana,* HMA4 von *Thlaspi caerulescens,* HMA4 von *Arabidopsis halleri,* subsp. *gemmifera,* HMA2 und HMA3 von *Oryza sativa* und HMA von *Vitis vinifera.*

5. Verfahren gemäß Anspruch 4, **dadurch gekennzeichnet, dass** die Variante einer P_{1B}-Typ ATPase die Aminosäuresequenz SEQ ID NO:2 hat.

6. Verfahren gemäß einem der Ansprüche 1 bis 5, **dadurch gekennzeichnet, dass** es weiter die Inhibition von mindestens einer endogenen P_{1B}-Typ ATPase der Zn²⁺/Co²⁺/Cd²⁺/Pb²⁺-Untergruppe, die das C₁PC₂-Motiv in der sechsten Transmembran-Domäne hat und *in planta* in der Plasmamembran lokalisiert ist umfasst.

7. Pflanzenzelle, **dadurch gekennzeichnet, dass** sie eine rekombinante Expressionskasette enthält, die ein Polynukleotid, das eine Variante einer P_{1B}-Typ ATPase, wie definiert in einem der Ansprüche 1 bis 5 kodiert, unter der Kontrolle eines transkriptionellen Promotors umfasst oder dadurch, dass sie einen rekombinanten Vektor enthält, der die rekombinante Expressionskasette enthält.

8. Pflanzenzelle gemäß Anspruch 7, **dadurch gekennzeichnet, dass** sie eine Tabakzelle ist.

9. Verfahren zur Herstellung einer transgenen Pflanze, die eine reduzierte Cadmium-Anhäufung hat, wohingegen Homöostase des physiologischen Zn²⁺-Mikronährstoffs unverändert ist, **dadurch gekennzeichnet, dass** es die folgenden Schritte umfasst:
a) Bereitstellen einer Pflanzenzelle gemäß Anspruch 7, und
b) Regenerieren einer transgenen Pflanze, die eine Variante einer P_{1B}-Typ ATPase, wie in einem der Ansprüche 1 bis 5 definiert exprimiert, aus der Pflanzenzelle erhalten in Schritt a).

10. Verfahren gemäß Anspruch 9, **dadurch gekennzeichnet, dass** die wie in Anspruch 6 definierte P_{1B}-Typ ATPase in der Pflanzenzelle, die in Schritt a) bereitgestellt wird, inhibiert ist.

11. Transgene Pflanze, die eine reduzierte Cadmium-Anhäufung hat, wohingegen Homöostase des physiologischen Zn²⁺-Mikronährstoffs unverändert ist, oder ein isoliertes Organ davon oder Gewebe davon, **dadurch gekennzeichnet, dass** sie/es, stabil in ihr/sein Genom integriert eine rekombinante Expressionskassette umfasst, die ein Polynukleotid, das eine Variante einer P_{1B}-Typ ATPase, wie definiert in einem der Ansprüche 1 bis 5 kodiert, unter der Kontrolle eines transkriptionellen Promotors umfasst.

12. Transgene Pflanze oder ein isoliertes Organ davon oder Gewebe davon gemäß Anspruch 11, **dadurch gekennzeichnet, dass** die wie in Anspruch 6 definierte endogene P_{1B}-Typ ATPase inhibiert ist.

13. Transgene Pflanze gemäß Anspruch 11 oder Anspruch 12, **dadurch gekennzeichnet, dass** sie eine transgene Tabakpflanze ist.

14. Isoliertes Organ gemäß Anspruch 11 oder Anspruch 12, **dadurch gekennzeichnet, dass** es ein Tabakblatt ist.

15. Verwendung eines Tabakblatts gemäß Anspruch 14 zur Herstellung von Tabakprodukten.

## Revendications

1. Procédé de réduction de l'accumulation de cadmium dans les parties aériennes d'une plante sans modification de l'homéostasie du nutriment physiologique Zn²⁺ dans ladite plante, **caractérisé en ce que** ledit procédé comprend l'expression dans ladite plante d'un variant d'une ATPase de type P_{1B} végétale du sous-groupe Zn²⁺ / Co²⁺ / Cd²⁺ / Pb²⁺ comprenant le motif C₁PC₂ dans le sixième domaine transmembranaire et localisé *in planta* au niveau de la membrane plasmique, ledit variant comprenant une mutation consistant en la substitution du résidu C₁ par un acide aminé quelconque choisi dans le groupe constitué d'une serine, une alanine, une histidine et une thréonine.

2. Procédé selon la revendication 1, **caractérisé en ce que** ladite ATPase de type P_{1B} est d'origine végétale supérieure.

3. Procédé selon la revendication 2, **caractérisé en ce que** ladite ATPase de type P_{1B} provient de la même espèce végétale que la plante pour laquelle ladite expression est souhaitée.

4. Procédé selon l'une quelconque des revendications 1 à 3, **caractérisé en ce que** ladite ATPase de type P_{1B} est choisie dans le groupe constitué par HMA4 et HMA2 d'*Arabidopsis thaliana,* HMA4 de *Thlaspi caerulescens,* HMA4 d'*Arabidopsis halleri* sous-espèce *gemmifera,* HMA2 et HMA3 d'*Oryza sativa* et HMA de *Vitis vinifera.*

5. Procédé selon la revendication 4, **caractérisé en ce que** le variant d'une ATPase de type P_{1B} a la séquence d'acides aminés SEQ ID No. : 2.

6. Procédé selon l'une quelconque des revendications 1 à 5, **caractérisé en ce que** ledit procédé comprend en outre l'inhibition d'au moins une ATPase de type P_{1B} endogène du sous-groupe Zn²⁺ / Co²⁺ / Cd²⁺ / Pb²⁺ comprenant le motif C₁PC₂ dans le sixième domaine transmembranaire et localisé *in planta* au niveau de la membrane plasmique.

7. Cellule végétale, **caractérisée en ce qu'**elle contient une cassette d'expression recombinante comprenant un polynucléotide codant pour un variant d'une ATPase de type P_{1B} tel que défini à l'une quelconque des revendications 1 à 5 sous le contrôle d'un promoteur de la transcription ou **caractérisée en ce qu'**elle contient un vecteur recombinant contenant ladite cassette d'expression recombinante.

8. Cellule végétale selon la revendication 7, **caractérisée en ce que** c'est une cellule de tabac.

9. Procédé de production d'une plante transgénique présentant une accumulation réduite de cadmium sans modification de l'homéostasie du micronutriment physiologique Zn²⁺, **caractérisé en ce qu'**il comprend les étapes suivantes :
a) fournir une cellule végétale selon la revendication 7, et
b) régénérer à partir de la cellule végétale obtenue à l'étape a) une plante transgénique exprimant un variant d'une ATPase de type P_{1B} tel que défini à l'une quelconque des revendications 1 à 5.

10. Procédé selon la revendication 9, **caractérisé en ce que** l'ATPase de type P_{1B} endogène telle que définie à la revendication 6 est inhibée dans ladite cellule végétale fournie à l'étape a).

11. Plante transgénique présentant une accumulation réduite de cadmium sans modification de l'homéostasie du micronutriment physiologique Zn²⁺ ou, organe isolé ou tissu de celle-ci, **caractérisés en ce qu'**ils comprennent, intégrée de manière stable dans son génome, une cassette d'expression recombinante comprenant un polynucléotide codant pour un variant d'une ATPase de type P_{1B} tel que défini à l'une quelconque des revendications 1 à 5, sous le contrôle d'un promoteur de la transcription.

12. Plante transgénique ou, organe isolé ou tissu de celle-ci selon la revendication 11, **caractérisés en ce que** l'ATPase de type P_{1B} endogène telle que définie à la revendication 6 est inhibée.

13. Plante transgénique selon la revendication 11 ou la revendication 12, **caractérisée en ce que** c'est une plante de tabac transgénique.

14. Organe isolé selon la revendication 11 ou la revendication 12, **caractérisé en ce que** c'est une feuille de tabac.

15. Utilisation d'une feuille de tabac selon la revendication 14 pour la fabrication de produits du tabac.


### SEQUENCE LISTING

<110> COMMISSARIAT A L'ENERGIE ATOMIQUE
   RICHAUD, Pierre
   GRAVOT, Antoine
   AUROY, Pascaline
   VAVASSEUR, Alain
<120> GENETICALLY MODIFIED PLANTS DISPLAYING REDUCED ACCUMULATION OF CADMIUM
<130> MJP/XRN/mad-F263/237-WO
<160> 2
<170> PatentIn version 3.3
<210> 1
   <211> 1172
   <212> PRT
   <213> Arabidopsis thaliana
<400> 1
   Met Ala Leu Gln Asn Lys Glu Glu Glu Lys Lys Lys Val Lys Lys Leu
   1               5                   10                  15
   Gln Lys Ser Tyr Phe Asp Val Leu Gly Ile Cys Cys Thr Ser Glu Val
               20                  25                  30
   Pro Ile Ile Glu Asn Ile Leu Lys Ser Leu Asp Gly Val Lys Glu Tyr
           35                  40                  45
   Ser Val Ile Val Pro Ser Arg Thr Val Ile Val Val His Asp Ser Leu
       50                  55                  60
   Leu Ile Ser Pro Phe Gln Ile Ala Lys Ala Leu Asn Glu Ala Arg Leu
   65                  70                  75                  80
   Glu Ala Asn Val Arg Val Asn Gly Glu Thr Ser Phe Lys Asn Lys Trp
                   85                  90                  95
   Pro Ser Pro Phe Ala Val Val Ser Gly Leu Leu Leu Leu Leu Ser Phe
               100                 105                 110
   Leu Lys Phe Val Tyr Ser Pro Leu Arg Trp Leu Ala Val Ala Ala Val
           115                 120                 125
   Ala Ala Gly Ile Tyr Pro Ile Leu Ala Lys Ala Phe Ala Ser Ile Lys
       130                 135                 140
   Arg Pro Arg Ile Asp Ile Asn Ile Leu Val Ile Ile Thr Val Ile Ala
   145                 150                 155                 160
   Thr Leu Ala Met Gln Asp Phe Met Glu Ala Ala Ala Val Val Phe Leu
                   165                 170                 175
   Phe Thr Ile Ser Asp Trp Leu Glu Thr Arg Ala Ser Tyr Lys Ala Thr
               180                 185                 190
   Ser Val Met Gln Ser Leu Met Ser Leu Ala Pro Gln Lys Ala Ile Ile
           195                 200                 205
   Ala Glu Thr Gly Glu Glu Val Glu Val Asp Glu Val Lys Val Asp Thr
       210                 215                 220
   Val Val Ala Val Lys Ala Gly Glu Thr Ile Pro Ile Asp Gly Ile Val
   225                 230                 235                 240
   Val Asp Gly Asn Cys Glu Val Asp Glu Lys Thr Leu Thr Gly Glu Ala
                   245                 250                 255
   Phe Pro Val Pro Lys Gln Arg Asp Ser Thr Val Trp Ala Gly Thr Ile
               260                 265                 270
   Asn Leu Asn Gly Tyr Ile Cys Val Lys Thr Thr Ser Leu Ala Gly Asp
           275                 280                 285
   Cys Val Val Ala Lys Met Ala Lys Leu Val Glu Glu Ala Gln Ser Ser
       290                 295                 300
   Lys Thr Lys Ser Gln Arg Leu Ile Asp Lys Cys Ser Gln Tyr Tyr Thr
   305                 310                 315                 320
   Pro Ala Ile Ile Leu Val Ser Ala Cys Val Ala Ile Val Pro Val Ile
                   325                 330                 335
   Met Lys Val His Asn Leu Lys His Trp Phe His Leu Ala Leu Val Val
               340                 345                 350
   Leu Val Ser Gly Cys Pro Cys Gly Leu Ile Leu Ser Thr Pro Val Ala
           355                 360                 365
   Thr Phe Cys Ala Leu Thr Lys Ala Ala Thr Ser Gly Leu Leu Ile Lys
       370                 375                 380
   Ser Ala Asp Tyr Leu Asp Thr Leu Ser Lys Ile Lys Ile Val Ala Phe
   385                 390                 395                 400
   Asp Lys Thr Gly Thr Ile Thr Arg Gly Glu Phe Ile Val Ile Asp Phe
                   405                 410                 415
   Lys Ser Leu Ser Arg Asp Ile Asn Leu Arg Ser Leu Leu Tyr Trp Val
               420                 425                 430
   Ser Ser Val Glu Ser Lys Ser Ser His Pro Met Ala Ala Thr Ile Val
           435                 440                 445
   Asp Tyr Ala Lys Ser Val Ser Val Glu Pro Arg Pro Glu Glu Val Glu
       450                 455                 460
   Asp Tyr Gln Asn Phe Pro Gly Glu Gly Ile Tyr Gly Lys Ile Asp Gly
   465                 470                 475                 480
   Asn Asp Ile Phe Ile Gly Asn Lys Lys Ile Ala Ser Arg Ala Gly Cys
                   485                 490                 495
   Ser Thr Val Pro Glu Ile Glu Val Asp Thr Lys Gly Gly Lys Thr Val
               500                 505                 510
   Gly Tyr Val Tyr Val Gly Glu Arg Leu Ala Gly Phe Phe Asn Leu Ser
           515                 520                 525
   Asp Ala Cys Arg Ser Gly Val Ser Gln Ala Met Ala Glu Leu Lys Ser
       530                 535                 540
   Leu Gly Ile Lys Thr Ala Met Leu Thr Gly Asp Asn Gln Ala Ala Ala
   545                 550                 555                 560
   Met His Ala Gln Glu Gln Leu Gly Asn Val Leu Asp Val Val His Gly
                   565                 570                 575
   Asp Leu Leu Pro Glu Asp Lys Ser Arg Ile Ile Gln Glu Phe Lys Lys
               580                 585                 590
   Glu Gly Pro Thr Ala Met Val Gly Asp Gly Val Asn Asp Ala Pro Ala
           595                 600                 605
   Leu Ala Thr Ala Asp Ile Gly Ile Ser Met Gly Ile Ser Gly Ser Ala
       610                 615                 620
   Leu Ala Thr Gln Thr Gly Asn Ile Ile Leu Met Ser Asn Asp Ile Arg
   625                 630                 635                 640
   Arg Ile Pro Gln Ala Val Lys Leu Ala Arg Arg Ala Arg Arg Lys Val
                   645                 650                 655
   Val Glu Asn Val Cys Leu Ser Ile Ile Leu Lys Ala Gly Ile Leu Ala
               660                 665                 670
   Leu Ala Phe Ala Gly His Pro Leu Ile Trp Ala Ala Val Leu Val Asp
           675                 680                 685
   Val Gly Thr Cys Leu Leu Val Ile Phe Asn Ser Met Leu Leu Leu Arg
       690                 695                 700
   Glu Lys Lys Lys Ile Gly Asn Lys Lys Cys Tyr Arg Ala Ser Thr Ser
   705                 710                 715                 720
   Lys Leu Asn Gly Arg Lys Leu Glu Gly Asp Asp Asp Tyr Val Val Asp
                   725                 730                 735
   Leu Glu Ala Gly Leu Leu Thr Lys Ser Gly Asn Gly Gln Cys Lys Ser
               740                 745                 750
   Ser Cys Cys Gly Asp Lys Lys Asn Gln Glu Asn Val Val Met Met Lys
           755                 760                 765
   Pro Ser Ser Lys Thr Ser Ser Asp His Ser His Pro Gly Cys Cys Gly
       770                 775                 780
   Asp Lys Lys Glu Glu Lys Val Lys Pro Leu Val Lys Asp Gly Cys Cys
   785                 790                 795                 800
   Ser Glu Lys Thr Arg Lys Ser Glu Gly Asp Met Val Ser Leu Ser Ser
                   805                 810                 815
   Cys Lys Lys Ser Ser His Val Lys His Asp Leu Lys Met Lys Gly Gly
               820                 825                 830
   Ser Gly Cys Cys Ala Ser Lys Asn Glu Lys Gly Lys Glu Val Val Ala
           835                 840                 845
   Lys Ser Cys Cys Glu Lys Pro Lys Gln Gln Val Glu Ser Val Gly Asp
       850                 855                 860
   Cys Lys Ser Gly His Cys Glu Lys Lys Lys Gln Ala Glu Asp Ile Val
   865                 870                 875                 880
   Val Pro Val Gln Ile Ile Gly His Ala Leu Thr His Val Glu Ile Glu
                   885                 890                 895
   Leu Gln Thr Lys Glu Thr Cys Lys Thr Ser Cys Cys Asp Ser Lys Glu
               900                 905                 910
   Lys Val Lys Glu Thr Gly Leu Leu Leu Ser Ser Glu Asn Thr Pro Tyr
           915                 920                 925
   Leu Glu Lys Gly Val Leu Ile Lys Asp Glu Gly Asn Cys Lys Ser Gly
       930                 935                 940
   Ser Glu Asn Met Gly Thr Val Lys Gln Ser Cys His Glu Lys Gly Cys
   945                 950                 955                 960
   Ser Asp Glu Lys Gln Thr Gly Glu Ile Thr Leu Ala Ser Glu Glu Glu
                   965                 970                 975
   Thr Asp Asp Gln Asp Cys Ser Ser Gly Cys Cys Val Asn Glu Gly Thr
               980                 985                 990
   Val Lys Gln Ser Phe Asp Glu Lys  Lys His Ser Val Leu  Val Glu Lys
           995                 1000                 1005
   Glu Gly  Leu Asp Met Glu Thr  Gly Phe Cys Cys Asp  Ala Lys Leu
       1010                 1015                 1020
   Val Cys  Cys Gly Asn Thr Glu  Gly Glu Val Lys Glu  Gln Cys Arg
       1025                 1030                 1035
   Leu Glu  Ile Lys Lys Glu Glu  His Cys Lys Ser Gly  Cys Cys Gly
       1040                 1045                 1050
   Glu Glu  Ile Gln Thr Gly Glu  Ile Thr Leu Val Ser  Glu Glu Glu
       1055                 1060                 1065
   Thr Glu  Ser Thr Asn Cys Ser  Thr Gly Cys Cys Val  Asp Lys Glu
       1070                 1075                 1080
   Glu Val  Thr Gln Thr Cys His  Glu Lys Pro Ala Ser  Leu Val Val
       1085                 1090                 1095
   Ser Gly  Leu Glu Val Lys Lys  Asp Glu His Cys Glu  Ser Ser His
       1100                 1105                 1110
   Arg Ala  Val Lys Val Glu Thr  Cys Cys Lys Val Lys  Ile Pro Glu
       1115                 1120                 1125
   Ala Cys  Ala Ser Lys Cys Arg  Asp Arg Ala Lys Arg  His Ser Gly
       1130                 1135                 1140
   Lys Ser Cys Cys Arg Ser Tyr Ala Lys Glu Leu Cys Ser His Arg
   1145 1150 1155
   His His His His His His His His His His His Val Ser Ala
   1160 1165 1170
<210> 2
   <211> 1172
   <212> PRT
   <213> Artificial
<220>
   <223> AtHMA4 variant
<220>
   <221> MISC_FEATURE
   <222> (357)..(357)
   <223> Xaa is selected from the group consisting of Ser, Ala, His and Thr
<400> 2
   Met Ala Leu Gln Asn Lys Glu Glu Glu Lys Lys Lys Val Lys Lys Leu
   1 5 10 15
   Gln Lys Ser Tyr Phe Asp Val Leu Gly Ile Cys Cys Thr Ser Glu Val
   20 25 30
   Pro Ile Ile Glu Asn Ile Leu Lys Ser Leu Asp Gly Val Lys Glu Tyr
   35 40 45
   Ser Val Ile Val Pro Ser Arg Thr Val Ile Val Val His Asp Ser Leu
   50 55 60
   Leu Ile Ser Pro Phe Gln Ile Ala Lys Ala Leu Asn Glu Ala Arg Leu
   65 70 75 80
   Glu Ala Asn Val Arg Val Asn Gly Glu Thr Ser Phe Lys Asn Lys Trp
   85 90 95
   Pro Ser Pro Phe Ala Val Val Ser Gly Leu Leu Leu Leu Leu Ser Phe
   100 105 110
   Leu Lys Phe Val Tyr Ser Pro Leu Arg Trp Leu Ala Val Ala Ala Val
   115 120 125
   Ala Ala Gly Ile Tyr Pro Ile Leu Ala Lys Ala Phe Ala Ser Ile Lys
   130 135 140
   Arg Pro Arg Ile Asp Ile Asn Ile Leu Val Ile Ile Thr Val Ile Ala
   145 150 155 160
   Thr Leu Ala Met Gln Asp Phe Met Glu Ala Ala Ala Val Val Phe Leu
   165 170 175
   Phe Thr Ile Ser Asp Trp Leu Glu Thr Arg Ala Ser Tyr Lys Ala Thr
   180 185 190
   Ser Val Met Gln Ser Leu Met Ser Leu Ala Pro Gln Lys Ala Ile Ile
           195                 200                 205
   Ala Glu Thr Gly Glu Glu Val Glu Val Asp Glu Val Lys Val Asp Thr
       210                 215                 220
   Val Val Ala Val Lys Ala Gly Glu Thr Ile Pro Ile Asp Gly Ile Val
   225                 230                 235                 240
   Val Asp Gly Asn Cys Glu Val Asp Glu Lys Thr Leu Thr Gly Glu Ala
                   245                 250                 255
   Phe Pro Val Pro Lys Gln Arg Asp Ser Thr Val Trp Ala Gly Thr Ile
               260                 265                 270
   Asn Leu Asn Gly Tyr Ile Cys Val Lys Thr Thr Ser Leu Ala Gly Asp
           275                 280                 285
   Cys Val Val Ala Lys Met Ala Lys Leu Val Glu Glu Ala Gln Ser Ser
       290                 295                 300
   Lys Thr Lys Ser Gln Arg Leu Ile Asp Lys Cys Ser Gln Tyr Tyr Thr
   305                 310                 315                 320
   Pro Ala Ile Ile Leu Val Ser Ala Cys Val Ala Ile Val Pro Val Ile
                   325                 330                 335
   Met Lys Val His Asn Leu Lys His Trp Phe His Leu Ala Leu Val Val
               340                 345                 350
   Leu Val Ser Gly Xaa Pro Cys Gly Leu Ile Leu Ser Thr Pro Val Ala
           355                 360                 365
   Thr Phe Cys Ala Leu Thr Lys Ala Ala Thr Ser Gly Leu Leu Ile Lys
       370                 375                 380
   Ser Ala Asp Tyr Leu Asp Thr Leu Ser Lys Ile Lys Ile Val Ala Phe
   385                 390                 395                 400
   Asp Lys Thr Gly Thr Ile Thr Arg Gly Glu Phe Ile Val Ile Asp Phe
                   405                 410                 415
   Lys Ser Leu Ser Arg Asp Ile Asn Leu Arg Ser Leu Leu Tyr Trp Val
               420                 425                 430
   Ser Ser Val Glu Ser Lys Ser Ser His Pro Met Ala Ala Thr Ile Val
           435                 440                 445
   Asp Tyr Ala Lys Ser Val Ser Val Glu Pro Arg Pro Glu Glu Val Glu
       450                 455                 460
   Asp Tyr Gln Asn Phe Pro Gly Glu Gly Ile Tyr Gly Lys Ile Asp Gly
   465                 470                 475                 480
   Asn Asp Ile Phe Ile Gly Asn Lys Lys Ile Ala Ser Arg Ala Gly Cys
                   485                 490                 495
   Ser Thr Val Pro Glu Ile Glu Val Asp Thr Lys Gly Gly Lys Thr Val
               500                 505                 510
   Gly Tyr Val Tyr Val Gly Glu Arg Leu Ala Gly Phe Phe Asn Leu Ser
   515 520 525
   Asp Ala Cys Arg Ser Gly Val Ser Gln Ala Met Ala Glu Leu Lys Ser
   530 535 540
   Leu Gly Ile Lys Thr Ala Met Leu Thr Gly Asp Asn Gln Ala Ala Ala
   545 550 555 560
   Met His Ala Gln Glu Gln Leu Gly Asn Val Leu Asp Val Val His Gly
   565 570 575
   Asp Leu Leu Pro Glu Asp Lys Ser Arg Ile Ile Gln Glu Phe Lys Lys
   580 585 590
   Glu Gly Pro Thr Ala Met Val Gly Asp Gly Val Asn Asp Ala Pro Ala
   595 600 605
   Leu Ala Thr Ala Asp Ile Gly Ile Ser Met Gly Ile Ser Gly Ser Ala
   610 615 620
   Leu Ala Thr Gln Thr Gly Asn Ile Ile Leu Met Ser Asn Asp Ile Arg
   625 630 635 640
   Arg Ile Pro Gln Ala Val Lys Leu Ala Arg Arg Ala Arg Arg Lys Val
   645 650 655
   Val Glu Asn Val Cys Leu Ser Ile Ile Leu Lys Ala Gly Ile Leu Ala
   660 665 670
   Leu Ala Phe Ala Gly His Pro Leu Ile Trp Ala Ala Val Leu Val Asp
   675 680 685
   Val Gly Thr Cys Leu Leu Val Ile Phe Asn Ser Met Leu Leu Leu Arg
   690 695 700
   Glu Lys Lys Lys Ile Gly Asn Lys Lys Cys Tyr Arg Ala Ser Thr Ser
   705 710 715 720
   Lys Leu Asn Gly Arg Lys Leu Glu Gly Asp Asp Asp Tyr Val Val Asp
   725 730 735
   Leu Glu Ala Gly Leu Leu Thr Lys Ser Gly Asn Gly Gln Cys Lys Ser
   740 745 750
   Ser Cys Cys Gly Asp Lys Lys Asn Gln Glu Asn Val Val Met Met Lys
   755 760 765
   Pro Ser Ser Lys Thr Ser Ser Asp His Ser His Pro Gly Cys Cys Gly
   770 775 780
   Asp Lys Lys Glu Glu Lys Val Lys Pro Leu Val Lys Asp Gly Cys Cys
   785 790 795 800
   Ser Glu Lys Thr Arg Lys Ser Glu Gly Asp Met Val Ser Leu Ser Ser
   805 810 815
   Cys Lys Lys Ser Ser His Val Lys His Asp Leu Lys Met Lys Gly Gly
   820 825 830
   Ser Gly Cys Cys Ala Ser Lys Asn Glu Lys Gly Lys Glu Val Val Ala
   835 840 845
   Lys Ser Cys Cys Glu Lys Pro Lys Gln Gln Val Glu Ser Val Gly Asp
   850 855 860
   Cys Lys Ser Gly His Cys Glu Lys Lys Lys Gln Ala Glu Asp Ile Val
   865 870 875 880
   Val Pro Val Gln Ile Ile Gly His Ala Leu Thr His Val Glu Ile Glu
   885 890 895
   Leu Gln Thr Lys Glu Thr Cys Lys Thr Ser Cys Cys Asp Ser Lys Glu
   900 905 910
   Lys Val Lys Glu Thr Gly Leu Leu Leu Ser Ser Glu Asn Thr Pro Tyr
   915 920 925
   Leu Glu Lys Gly Val Leu Ile Lys Asp Glu Gly Asn Cys Lys Ser Gly
   930 935 940
   Ser Glu Asn Met Gly Thr Val Lys Gln Ser Cys His Glu Lys Gly Cys
   945 950 955 960
   Ser Asp Glu Lys Gln Thr Gly Glu Ile Thr Leu Ala Ser Glu Glu Glu
   965 970 975
   Thr Asp Asp Gln Asp Cys Ser Ser Gly Cys Cys Val Asn Glu Gly Thr
   980 985 990
   Val Lys Gln Ser Phe Asp Glu Lys Lys His Ser Val Leu Val Glu Lys
   995 1000 1005
   Glu Gly Leu Asp Met Glu Thr Gly Phe Cys Cys Asp Ala Lys Leu
   1010 1015 1020
   Val Cys Cys Gly Asn Thr Glu Gly Glu Val Lys Glu Gln Cys Arg
   1025 1030 1035
   Leu Glu Ile Lys Lys Glu Glu His Cys Lys Ser Gly Cys Cys Gly
   1040 1045 1050
   Glu Glu Ile Gln Thr Gly Glu Ile Thr Leu Val Ser Glu Glu Glu
   1055 1060 1065
   Thr Glu Ser Thr Asn Cys Ser Thr Gly Cys Cys Val Asp Lys Glu
   1070 1075 1080
   Glu Val Thr Gln Thr Cys His Glu Lys Pro Ala Ser Leu Val Val
   1085 1090 1095
   Ser Gly Leu Glu Val Lys Lys Asp Glu His Cys Glu Ser Ser His
   1100 1105 1110
   Arg Ala Val Lys Val Glu Thr Cys Cys Lys Val Lys Ile Pro Glu
   1115 1120 1125
   Ala Cys Ala Ser Lys Cys Arg Asp Arg Ala Lys Arg His Ser Gly
   1130 1135 1140
   Lys Ser  Cys Cys Arg Ser Tyr  Ala Lys Glu Leu Cys  Ser His Arg
       1145                 1150                 1155
   His His  His His His His His  His His His His Val  Ser Ala
       1160                 1165                 1170